# EUROPEAN PATENT APPLICATION

(11) **EP 4 273 063 A1**
(43) Date of publication of application: **08.11.2023**
(21) Application number: 23168868.0
(22) Date of filing: 20.04.2023
(51) Int. Cl.: B65D 25/10, A61M 5/00, B01L 9/00, B65D 71/70, F26B 5/06

(54) **HOLDER DEVICE FOR HOLDING A PLURALITY OF VIALS TO BE MOVED IN A PROCESSING LINE**

(30) Priority: 02.05.2022 IT 202200008831
(71) Applicant: NUOVA OMPI S.r.l. Unipersonale, 35017 Piombino Dese (PD) (IT)
(72) Inventor: CANESTRARO, Marco, 35010 Carmignano di Brenta (PD) (IT); FRARE, Maria Chiara, 35027 Noventa Padovana (PD) (IT); ZANETTI, Francesco, 30033 Noale (VE) (IT)
(74) Representative: Porta & Consulenti Associati S.p.A.

(57) **Abstract**

The invention relates to a holder device for holding (10) a plurality of vials (50) to be moved in a processing line. The holder device (10) comprises a plate (20) having a plurality of housing seats (30) for housing the vials (50) and a plurality of rigid spacer elements (40) arranged within the housing seats (30) and configured to maintain the vials (50) in a suspended position with respect to a substantially horizontal operating surface (100) of the processing line. Each housing seat (30) comprises an open base (32) intended to face towards the operating surface (100), the open base (32) being distinct from the spacer elements (40).

## Description

The present invention relates to a device for holding a plurality of vials to be moved in a processing line.

This holder device meets the definition of "nest" of standard ISO 21882: 2019.

The present invention also relates to an organised assembly of vials comprising the aforementioned holder device and a plurality of vials held by said holder device.

The present invention also relates to a container for housing the aforementioned organised assembly of vials.

Preferably, the vials which this description and the subsequent claims refer to are vials for pharmaceutical use made of glass or thermoplastic material.

According to ISO 21882:2019, the nest is used to safely move a plurality of vials along processing lines and typically comprises a plate made of a plastic material on which a plurality of seats for housing the vials are formed. These housing seats are shaped so as to hold the various vials in a stable manner, avoiding possible mutual collisions during the movement of the nest.

Typically, several process steps are actuated in the aforesaid processing lines, such as the sterilisation of empty vials, filling of vials and storage of filled vials.

In many cases, a freeze-drying step is also carried out in the processing lines, which comprises at first freezing the liquid product contained in the vials and then reducing the pressure and introducing heat to allow the water frozen in the product which is inside the vials to sublime.

Pharmaceutical companies often carry out the freeze-drying step because it allows the quality of the product inside the vials to be maintained unchanged and, thus, the shelf life of the product to increase. Freeze-drying also makes it easier moving the vials in the processing line after they have been filled.

The freeze-drying step has some critical issues.

A first critical issue is related to the need to provide for a homogeneous heat transfer within the vial from the heating surface of the freeze-drying chamber.

A second critical issue is related to the fact that freeze-drying takes a long time, up to 300 hours to complete one cycle.

A further critical issue is related to the fact that pharmaceutical products are often freeze-dried in large batches, which can lead to variations in the quality of the freeze-dried product depending on the position of the vial in the freeze-drying chamber.

Therefore, thoughts have been made about how to carry out fast freeze-drying steps leading to homogeneous freeze-dried products regardless of the position of the vials in the freeze-drying chamber.

From publication "From Batch to Continuous: Freeze-Drying of Suspended Vials for Pharmaceuticals in Unit-Doses - Luigi C. Capozzi, Bernhardt L. Trout, and Roberto Pisano - Industrial & Engineering Chemistry Research 2019 58 (4), 1635-1649 - DOI: 10.1021/acs.iecr.8b02886" it is known to carry out the freeze-drying step on vials that are kept suspended from the heating surface of the freeze-drying chamber. This enables an uniform transfer of heat from the heating surface to all the vials provided in the freeze-drying chamber and a processing time 3-4 times shorter than if the vials were resting on the heating surface.

From publication "Harguindeguy M, Stratta L, Fissore D, Pisano R. Investigation of the Freezing Phenomenon in Vials Using an Infrared Camera. Pharmaceutics. 2021 Oct 12;13(10):1664. doi: 10.3390/ pharmaceutics13101664. PMID: 34683957; PMCID: PMC8539506" it is known that, in the case of suspended vials, heat is transferred evenly from the heating surface to the inside of the vials mainly by natural convection through the base and the side surface of the vials.

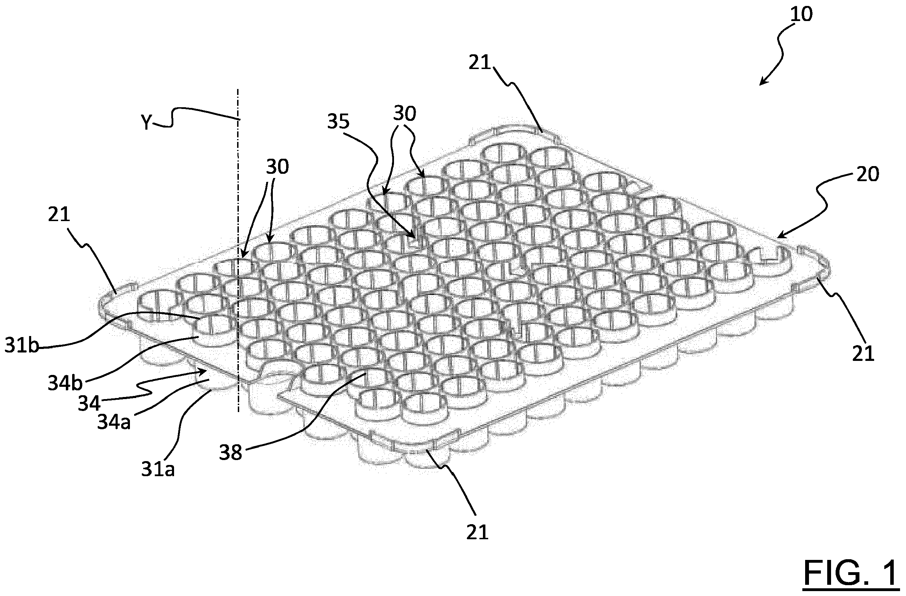

WO 2016075647 A1 describes freeze-drying processes in which vials housed in a holder device are not in contact with the heating surface. In particular, in variant 3 of Figure 1 of WO 2016075647 A1 spacers made of a thermally insulating material are used. These spacers are placed between the heating surface and the holder device preferably below the bases of some housing seats arranged at the edges of the holder device or between pairs of adjacent housing seats. Differently, in variant 4 of figure 1 the holder device keeps the vials suspended from the heating surface by holding them at the respective necks.

The Applicant believes that the solution of variant 3 has some drawbacks.

Firstly, the spacers may collapse, deform and/or move with respect to the heating surface and/or the holder device, e.g. even just due to the force which is applied in order to close the vials, with a consequent negative impact on the freeze-drying step. If even just one spacer is defective or damaged or moves, the vials may no longer all stand at the same distance from the heating surface, and the freeze-dried product inside the vials of the same production batch may not be homogeneous.

In order to limit the negative impact caused by possible failure, deformation and/or displacement of spacers, it would be necessary to increase the number of spacers used, with obvious impact on cost.

The use of spacers is also expensive due to the fact that they have to be manufactured, typically by moulding, and thus a certain amount of moulding material has to be purchased.

The Applicant also noted that the use of a thermally insulating material to manufacture the spacers may adversely affect the transfer of heat from the heating surface to the inside of the vials.

Furthermore, the use of spacers is not a functional and easily implementable solution in industrial processes, where there is a need for high automation and large production batches.

Additionally, the bases of the housing seats are closed. This does not allow an optimal heat exchange between the heating surface and the vials, with a consequent negative effect on the result of the freeze-drying step.

According to the Applicant, the solution of the aforementioned variant 4, on the other hand, has the disadvantage that the vials can be damaged or break at the respective necks during the vial closing step, since this closing step involves the application of a high pressure on the vials.

The technical problem underlying the present invention is to provide a holder device for holding a plurality of vials to be moved in a processing line that does not have the drawbacks discussed above.

WO 2016166765 A1 discloses a supporting device for supporting a plurality of sealed medical cartridges, comprising a supporting plate having a plurality of receptacles for accommodating the cartridges. Protrusions are formed at the bottom ends of the receptacles, the protrusions protruding inward and supporting shoulder portions of the cartridges in such a manner that the upper ends of the cartridges protrude from the upper ends of the receptacles when the cartridges are accommodated upside-down in the receptacles.

US 2003217947 A1 discloses a supporting device for supporting an article, including a main body and at least one deformable supporting element disposed in the main body.

WO 2014130349 A1 discloses a supporting device for supporting a plurality of vials, including a substantially planar plate and a plurality of vial housing seats defined in the plate.

The present invention thus relates, in a first aspect, to a holder device for holding a plurality of vials to be moved in a processing line according to claim 1.

In this description and the appended claims, the term "suspended position" is used to indicate a position such that between the vials and the operating surface of the processing line on which the holder device is placed there is a gap such that heat is exchanged by natural convection (and not by conduction) between said operating surface and the vials.

The provision of housing seats having respective open bases facing the operating surface of the processing line and of spacer elements within the housing seats makes it possible to achieve ideal conditions for carrying out a freeze-drying step which is suitable for guaranteeing the desired quality and homogeneity of the freeze-dried product inside the various vials of the same production batch. In this way, in fact, each vial is supported in a suspended position with respect to the aforementioned operating surface independently of the other vials and the heat coming from the operating surface can reach the vials directly, passing through the open bases of the housing seats.

The spacer elements can be made as a single piece with the plate, achieving clear benefits with regard to production costs and industrial processability with respect to solutions involving spacers arranged outside the housing and made as separate pieces from the plate, such as Variant 3 of WO 2016075647 A1.

The bases of the housing seats allow for a stable support of the vials also during the closing steps of the vials, eliminating the risk of breakage and/or damage typical of those solutions where the vials are gripped at the respective necks, such as in variant 4 of WO 2016075647 A1.

In a second aspect thereof, the present invention relates to an organised assembly of vials according to claim 11.

Such an assembly allows simultaneous processing of all the vials of a production batch along the processing line.

In a third aspect thereof, the present invention relates to a container for housing an organised assembly of vials according to the second aspect of the present invention, wherein each of said vials contains a freeze-dried product.

This container allows simultaneous movement of the vials of a production batch along the processing line.

Preferred features of the invention are described below. Each of such features can be provided individually or in combination with the other preferred features.

Preferably, said base defines a first support plane for supporting the holder device on said operating surface.

Preferably, at least one of said spacer elements defines, within the housing seat, a second support plane for supporting the vial.

Preferably, said second support plane is parallel to said first support plane and is spaced apart from said first support plane by a predetermined distance.

In preferred embodiments, said predetermined distance is greater than three times said predetermined thickness, preferably equal to four times said predetermined thickness. This distance ensures the vial to be suspended with respect to the operating surface at a height that the Applicant deems optimal for an efficient freeze-drying step.

Preferably, each housing seat comprises a tubular wall extending from said base along a substantially vertical longitudinal axis.

Preferably, each housing seat comprises at least three spacer elements radially protruding from said tubular wall towards the inside of said housing seat.

Preferably, said at least three spacer elements are circumferentially equally spaced apart from each other.

In some preferred embodiments, there are four spacer elements and they are angularly offset by 90°.

In particularly preferred embodiments, there are six spacer elements and they are angularly offset by 60°.

In the present description and the appended claims, the term "axial" and the corresponding term "axially" are used to refer to the longitudinal direction of the housing seat of the holder device, the term "radial" and the corresponding term "radially" are used to refer to any direction perpendicular to the aforementioned longitudinal direction, and the term "circumferential" and the corresponding term "circumferentially" are used to refer to any circumferential direction around the aforementioned longitudinal direction.

The provision of at least three circumferentially equally spaced apart spacer elements ensures an optimal and stable support of the vials in the respective housing seats.

In a preferred embodiment, the spacer elements are formed in a single piece with said tubular wall, and thus with said plate. In this way, the holder device can be manufactured by moulding a plastic material, without the need to provide for separate elements with respect to the plate.

In an alternative embodiment, the spacer elements are made as separate pieces from the housing seats and can be housed, possibly removably, in the respective housing seats.

In that case, preferably, each of said spacer elements comprises an annular body and a plurality of appendages extending from the annular body, said appendages being configured to support a vial.

More preferably, said annular body comprises at least one anti-rotational element configured to prevent the annular body from freely rotating about the longitudinal axis of the housing seat after the spacer element is arranged within said housing seat.

In all the embodiments discussed above, preferably, said base is defined by at least three support elements radially protruding from said tubular wall towards the inside of said housing seat and circumferentially equally spaced apart from each other.

In some embodiments, there are four support elements and they are angularly offset by 90°.

In particularly preferred embodiments, there are six support elements and they are angularly offset by 60°.

In a preferred embodiment, each spacer element is arranged above a respective support element. In this case, the latter acts both as a reinforcing element of the respective spacer element and as a reinforcing element of the plate of the holder device.

Preferably, the support elements are formed in a single piece with said tubular wall, with obvious benefits with regard to the ease of production and cost.

Preferably, the spacer elements have an axial extension larger than that of the support elements. The axial extension of the spacer elements is also chosen depending on that of the support elements in order to place the vial bases at the predetermined distance from the operating surface.

Preferably, the spacer elements have a circumferential extension smaller than that of the support elements. In this way, the contact area between the vials and the spacer elements is reduced, so that the heat exchange between the operating surface and the vials during the freeze-drying step is facilitated.

Preferably, the spacer elements have a radial extension smaller than that the support elements. This ensures a stable support of the vials within the housing while guaranteeing the desired heat exchange between the operating surface and the vials.

Preferably, each housing seat comprises at least three longitudinal ribs radially protruding from said tubular wall towards the inside of the housing seat. These longitudinal ribs help to stiffen the plate of the holder device.

Preferably, all the longitudinal ribs have the same shape and size and are circumferentially equally spaced apart from each other, so as to achieve a precise and centred positioning of the vials within the housing seats.

Preferably, each of said at least three spacer elements is an extension of a respective longitudinal rib. This allows for a better distribution of the vertical load applied on the holder device and the vials when the latter are being closed.

Preferably, each housing seat comprises a longitudinal rib protruding radially from the tubular wall towards the outside of said housing seat and connected to the tubular wall of an adjacent housing seat. These longitudinal ribs give rigidity to the tubular walls of the housing seats and allow a better distribution of the vertical load applied for example when the vials are being closed.

Preferably, each longitudinal rib is arranged at a respective longitudinal rib.

Preferably, the housing seats extend on opposite sides with respect to said plate.

Preferably, the tubular walls of some pairs of adjacent housing seats comprise, on the opposite side of the respective base with respect to the said plate, respective mutually facing lateral through openings. These lateral through openings allow the positioning on the plate of additional devices used in certain processing steps in combination with the holder device, such as vial-closing devices.

Preferably, the spacer elements are made of a material that is at least partially thermally conductive or not thermally insulating, so that the heat exchange between the operating surface and the vials during the freeze-drying step is facilitated or not hindered.

Further characteristics and advantages of the present invention will become clear from the following detailed description of preferred embodiments thereof, made with reference to the appended drawings and provided for indicative and non-limiting purposes. In such drawings:
- Figure 1 is a schematic perspective view from above of a first embodiment of a holder device according to the present invention;
- Figure 2 is a schematic perspective view from below of the holder device of Figure 1;
- Figure 3 is an enlarged schematic plan view of a portion of the holder device of Figure 1;
- Figure 4 is an enlarged schematic perspective view of a detail of the portion of Figure 3;
- Figure 5 is an enlarged schematic perspective view of a further portion of the holder device of figure 1;
- Figure 6 is an enlarged schematic perspective view of a cross-section of a portion of the holder device of Figure 1 and of a vial held by that holder device;
- Figure 7 is an enlarged schematic perspective view of a cross-section of a portion of a second embodiment of the holder device of the invention and of a vial held by said holder device;
- Figure 8 is an enlarged schematic perspective view of a cross-section of a component of the holder device of Figure 7;
- Figure 9 is a schematic perspective view of a container of a holder device according to the present invention.

A first embodiment of a holder device 10 in accordance with the present invention is shown in Figures 1-6.

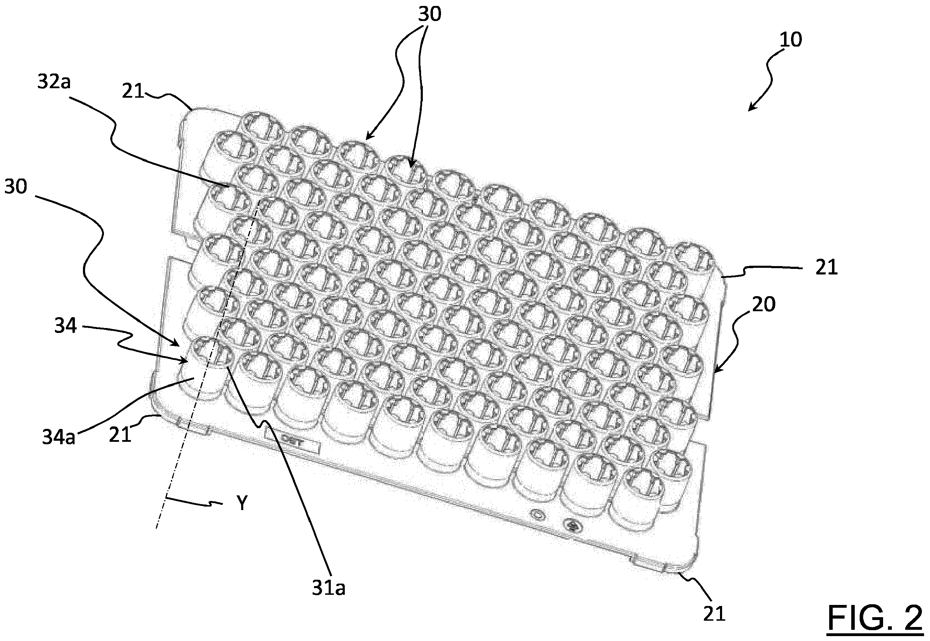

The holder device 10 comprises a square-shaped plate 20. In the non-limiting example of Figures 1 and 2, the plate 20 is substantially rectangular and has four rounded vertices 21.

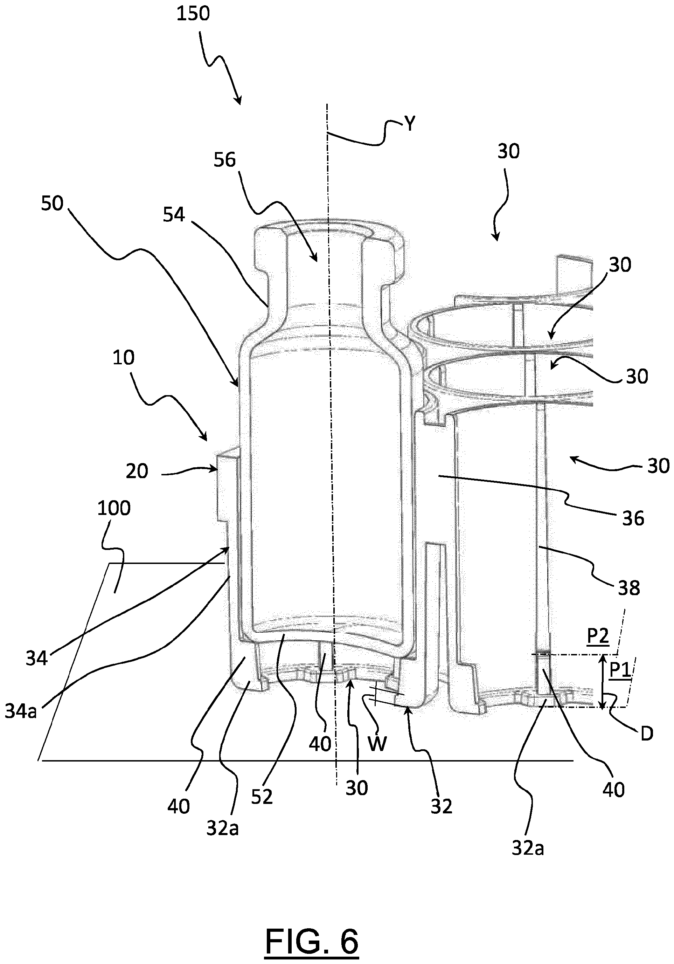

The plate 20 comprises a plurality of housing seats 30 for housing respective vials 50 (Figure 6).

Each vial 50 has a substantially cylindrical shape and comprises a substantially circular closed base surface 52 and, on the opposite side to the base surface 52, a neck 54 provided with a through opening 56 to allow the vial 50 to be filled with a predetermined amount of a pharmaceutical product.

The vials 50 are made of glass or of a thermoplastic material.

Upon having arranged the vials 50 of a production batch in the housing seats 30 of the holder device 10 an organised assembly 150 of vials 50 is obtained that allows simultaneous processing of all the vials 50 of the production batch, such as carrying out a freeze-drying step of the pharmaceutical product provided in the vials 50. The vials 50 can be filled either before or after the vials 50 are placed in the respective housing seats 30.

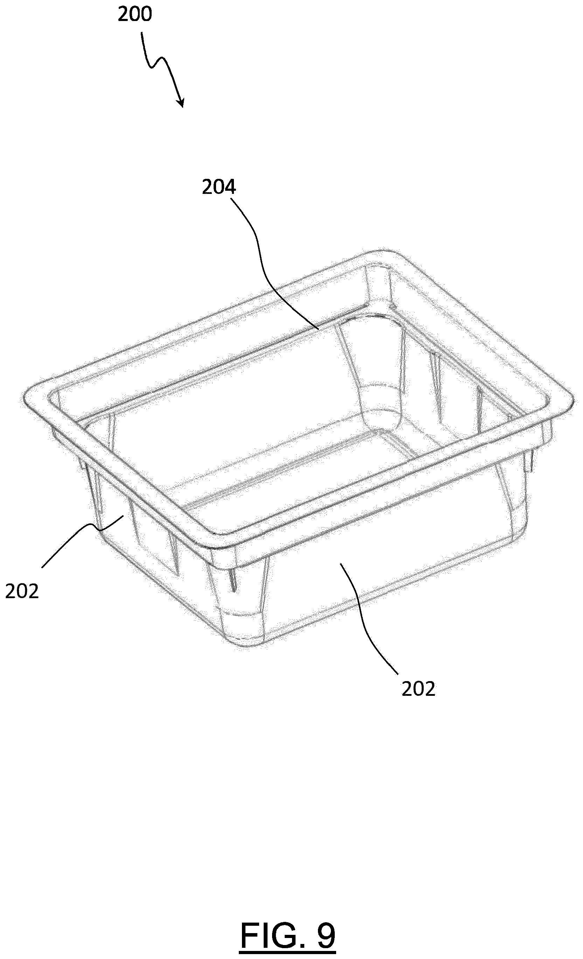

The vials 50 are moved along a processing line (not shown) and, optionally, stored by arranging the holder device 10 in a container 200 shown in Figure 9.

In the non-limiting example of Figure 9, the container 200 has a substantially parallelepiped or truncated pyramid shape and comprises four side walls 202. An abutment surface 204, on which the plate 20 of the holder device 10 is intended to be supported, is formed on the inner walls of the side walls 202.

In this way, the vials 50 of a production batch are aggregated in a so-called "nest and tub" configuration, in which the holder device 10 is the "nest" and the container 200 is the "tub".

The processing steps which the vials 50 housed in the housing seats 30 might be subjected to can be for example sterilisation, filling, freeze-drying, closure, etc. or manual and/or automated inspection operations, etc.

In the plate 20, the housing seats 30 are arranged with respect to each other according to a predetermined geometric configuration.

In the non-limiting example of Figures 1-6, the housing seats 30 are arranged in a hexagonal configuration, i.e. wherein each housing seat 30 other than the housing seats 30 located at the edges of the plate 20 is surrounded by six housing seats 30.

Each of the housing seats 30 comprises, at a lower end 31a thereof, a base 32 having a through opening 33 and, at an upper end 31b thereof, a through opening configured to allow insertion and removal of a vial 50.

The base 32 has a predetermined thickness W (Figure 6).

The base 32 is intended to rest on a substantially horizontal operating surface 100 of the processing line, e.g. the heating surface of a temperature-controlled shelf of a freeze-drying chamber.

The bases 32 of the housing seat 30 define a support plane P1 of the plate 20 on the operating surface 100 (figure 6).

In this description and the appended claims, the terms "below", "lower" and "downwardly", "above", "upper" and "upwardly", referred to the holder device 10 or parts thereof, are used with reference to the position assumed by the holder device 10 when it rests on the operating surface 100.

Each housing 30 also comprises a tubular wall 34 which, in the non-limiting example of Figures 1-6, is substantially cylindrical.

The tubular wall 34 extends above the base 32 along a substantially vertical longitudinal axis Y.

The vials 50 are arranged in the respective housing seats 30 so that their axis substantially coincides with the longitudinal axis Y.

As shown in Figures 1 and 2, the housing seats 30 extend on opposite sides with respect to the plate 20. In this way, a lower portion 34a of the tubular wall 34 is placed below the plate 20 (i.e. on the side of the base 32) and an upper portion 34b of the tubular wall 34 is placed above the plate 20 (i.e. on the side of the upper end 31b).

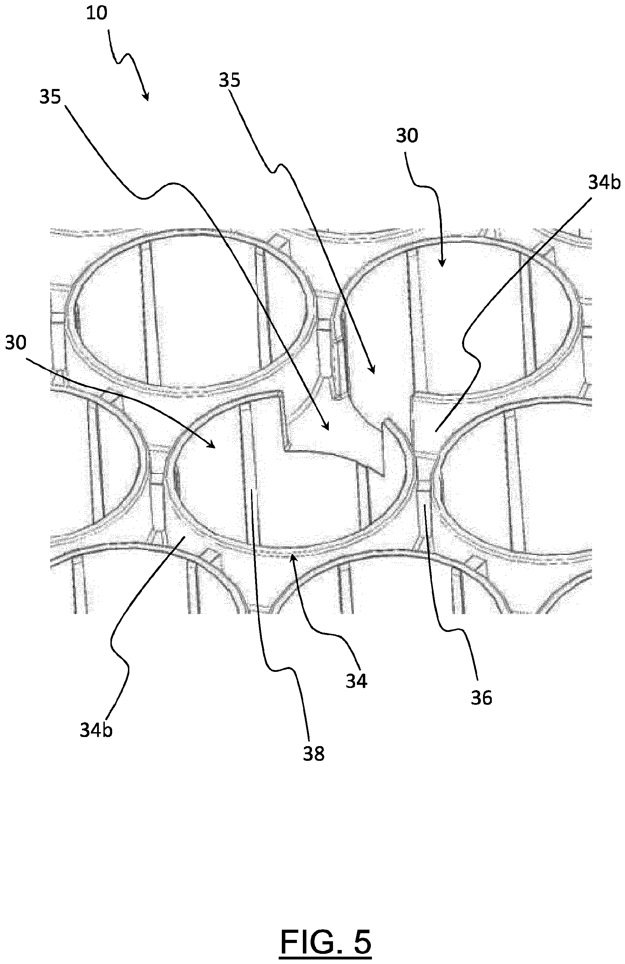

As shown in Figures 1 and 5, the upper portions 34b of the tubular walls 34 of some pairs of adjacent housing seats 30 comprise mutually facing lateral through openings 35. These lateral through openings 35 extend up to the upper end 31b of the housing seats 30.

In the non-limiting example of Figures 1-6, the base 32 is defined by six support elements 32a radially protruding from the tubular wall 34 towards the inside of the housing seat 30 and circumferentially equidistant from each other, i.e. angularly offset by 60° about the longitudinal axis Y.

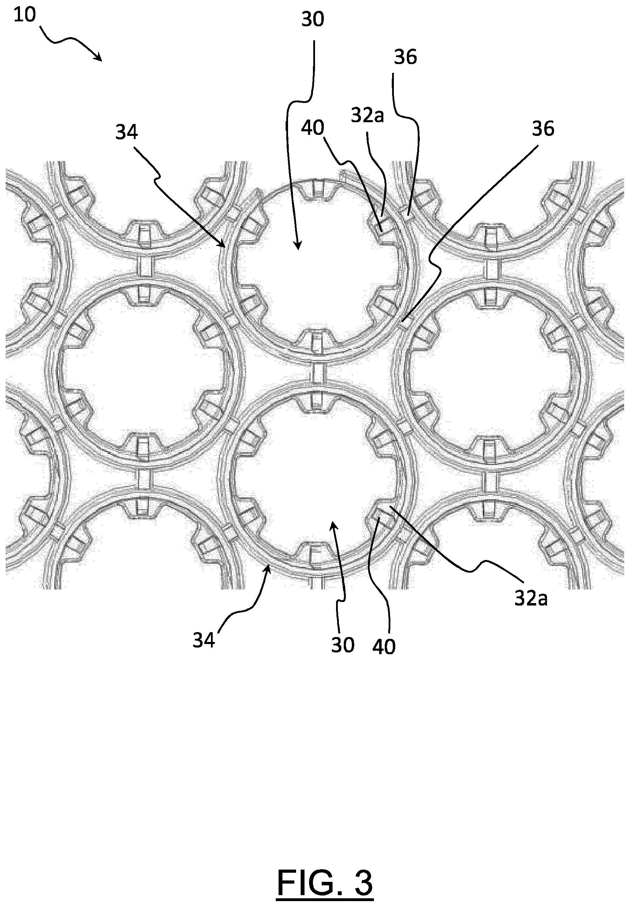

As shown in Figures 3-6, a longitudinal rib 36 extends externally to each housing seat 30 from the respective tubular wall 34 to the tubular wall 34 of an adjacent housing seat 30 along a substantially radial direction so as to connect the two housing seats 30.

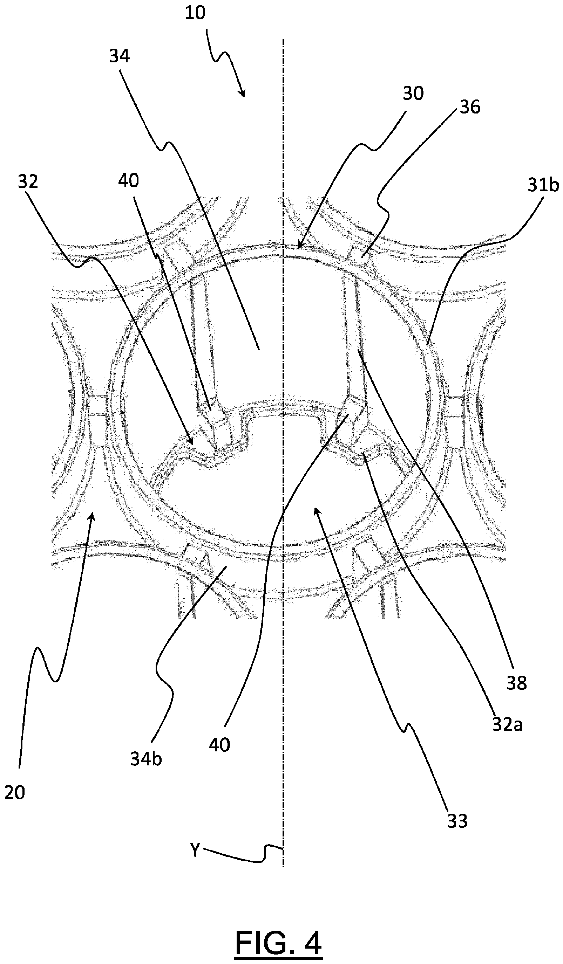

A plurality of longitudinal ribs 38 extend internally to each housing seat 30 and protrude radially from the tubular wall 34. In the non-limiting example of Figures 4-6, there are six longitudinal ribs 38, which are identical in shape and size, circumferentially equally spaced apart from each other, located above the support elements 32a and each at a respective longitudinal rib 36.

A plurality of rigid spacer elements 40 is provided within each housing seat 30.

In the non-limiting example of Figures 1-6, each housing seat 30 comprises six spacer elements 40 radially protruding from the tubular wall 34 towards the inside of the housing seat 30 and circumferentially equally spaced apart from each other, i.e. arranged angularly offset by 60° about the longitudinal axis Y.

The spacer elements 40 define inside the housing seat 30 a support plane P2 for the vial 50 (Figure 6).

This support plane P2 is parallel to the support plane P1 and is spaced apart from the first support plane by a predetermined distance D.

This distance D is preferably greater than three times the predetermined thickness W of the base 32, for example four times the predetermined thickness W.

In the non-limiting example of Figures 4 and 6, each spacer element 40 is arranged above a respective support element 32a and the longitudinal ribs 38 are arranged above the spacer elements 40. In this way, each spacer element 40 can be made in a single piece with the respective support element 32a and the respective longitudinal rib 38 and defines an extension or continuation of the latter.

In the non-limiting example of Figures 1-6, the spacer elements 40, the support elements 32a, the longitudinal ribs 38 and the longitudinal ribs 36 are formed in a single piece with the tubular wall 34 and thus with the plate 20.

In the non-limiting example of Figures 3 and 4, the spacer elements 40 have a circumferential extension smaller than that of the respective support elements 32a.

Furthermore, the spacer elements 40 have a axial extension larger than that of the respective support elements 32a. This axial extension is for example comprised between 0.1 mm and 12 mm, preferably between 0.1 mm and 10 mm, the extreme values being included.

For example, the thickness (or axial extension) W of the base 32 is comprised between 0.1 mm and 4 mm, the extreme values being included.

Preferably, the spacer elements 40 have an axial extension substantially equal to three times the thickness W of the base 32.

Furthermore, the spacer elements 40 have a radial extension smaller than that of the respective support elements 32a.

This radial extension, calculated at the support plane P2, has a value chosen depending on the diameter of the base surface 52 of the vial 50, with a maximum value equal to the inner radius of the housing seat 30.

The base surface 52 of the vial 50 has a diameter larger than the one which is defined by the radially inner ends of the spacer elements 40.

The holder device 10 described above can be made by moulding a plastic material, preferably a partially thermally conductive and non-thermally insulating plastic material, e.g. polypropylene.

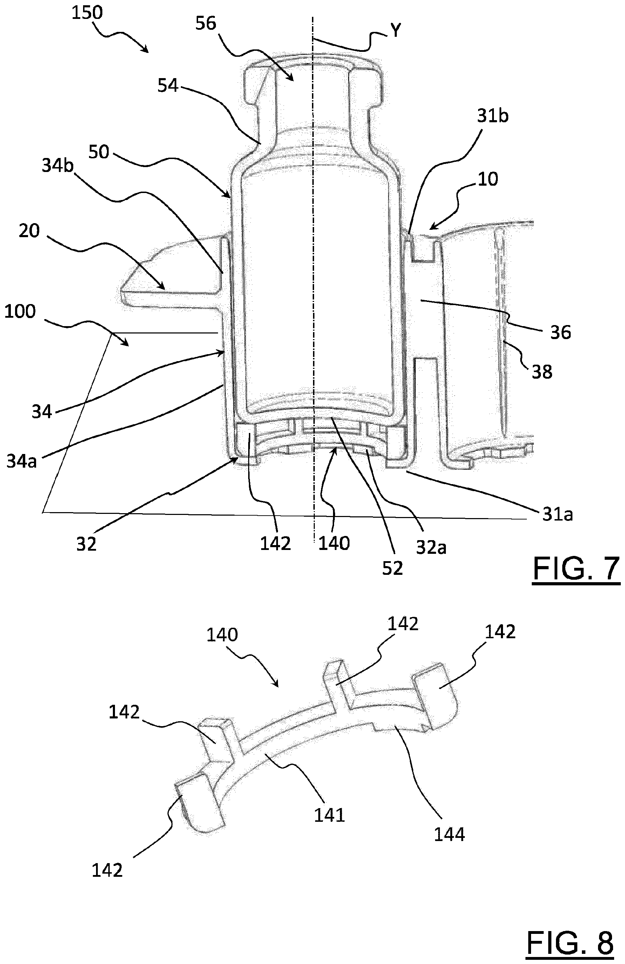

Figures 7 and 8 show a second embodiment of a holder device 10 according to the present invention. Parts and components that are identical or similar to those of the holder device 10 of Figures 1-6 are given the same reference number and for their description reference can be made to the discussion above.

The holder device 10 of Figures 7 and 8 differs from the holder device 10 of Figures 1-6 only in the spacer elements 140, which in this case are made as separate pieces from the plate 20. The spacer elements 140 are inserted into the respective housing seats 30 and optionally can be removed from the housing seats 30.

Each spacer element 140 of Figures 7 and 8 comprises an annular body 141 and six appendages 142 extending axially above the annular body 141. In Figure 8, the spacer element 140 is sectioned in half, so that two whole appendages 142 and two opposing appendages 142 which are sectioned in half are shown.

The appendages 142 are circumferentially equally spaced apart from each other, i.e. they are angularly offset by 60° around the longitudinal axis Y, and have a conformation similar to that of spacer elements 40 of Figures 1-6.

Referring to Figure 7, the spacer element 140 is inserted into the housing seat 30 from the upper end 31b until the annular body 141 rests on the support elements 32a. The appendages 142 radially protrude from the tubular wall 34 towards the inside of the housing seat 30.

Each appendage 142 is arranged above a respective support element 32a.

Similar to the spacer elements 40 of Figures 1-6, each appendage 142 has a circumferential extension preferably smaller than that of the support element 32a, an axial extension preferably larger than that of the support element 32a, and a radial extension preferably smaller than that of the support element 32a.

The spacer element 140 of Figures 7 and 8 further comprises at least one anti-rotational element 144 which extends axially below the annular body 141. The anti-rotational element 144 abuts against one of the support elements 32a (or against a specially provided contrast element), preventing the spacer element 140 from rotating freely about the longitudinal axis Y.

The anti-rotational element 144 has an axial extension smaller than or equal to that of the support elements 32a.

The Applicant verified by means of experimental tests and finite element simulations (FEM) that the holder device 10 of the invention ensures the integrity of the vials during the closing thereof, which typically occurs after the freeze-drying step. This is despite the fact that the mechanical strength of the vials is reduced due to the freeze-drying step itself.

The Applicant also verified that the provision of the spacer elements 40, 140 in the holder device 10 of the invention does not influence the strength of the holder device 10 in the different steps of the processing line.

In particular, the Applicant carried out a finite element simulation (FEM) by subjecting to a vertical compressive stress similar to that exerted during the closing of the vials both vials arranged in the holder device 10 of the invention and vials arranged in a conventional nest, without spacer elements 40, 140.

Through experimental tests, it was determined that a vial resting on a surface and subjected to vertical compression breaks at 1100 N, corresponding to a breaking load of 230 MPa, and that the force applied to close a vial with a cap which seals internally the vial is 5 N while the force applied to close a vial with a cap sealing externally the vial is 100 N.

The most onerous case of a cap sealing externally a vial was considered. Such a cap was coupled to a vial arranged in the holder device 10 of the invention and to a vial arranged in a conventional nest.

The stress applied to the vial arranged in the conventional nest was 34 MPa while the force applied to the vial arranged in the holder device 10 of the invention was 92 MPa, and thus well below the breaking load of 230 MPa.

The stress generated in the holder device 10 of the invention was also measured, to verify that the deformation of the plastic material (typically polypropylene) did not lead to damage (i.e. permanent deformation).

It is known from the data sheet of polypropylene that this material remains in the linear deformation range when the deformation is less than 10%.

The results obtained through simulations with the conventional nest and the holder device 10 of the invention were both below the 10% threshold value. For the conventional nest, a maximum value of 5% was measured and for the holder device 10 of the invention a maximum value of 7% was measured. This demonstrated that in the holder device 10 of the invention no permanent deformations are generated during the closing of the vials.

Obviously, in order to meet specific and contingent requirements, a person skilled in the art can make several changes and variations to the above-described invention, all of which are contained within the scope of protection defined by the following claims.

In a non-shown variant of the first embodiment of the holder device 10 of Figures 1-6, the spacer elements 40 could be circumferentially connected to each other to form a single annular structure similar to the spacer element 140 of Figures 7 and 8.

## Claims

1. A holder device (10) for holding a plurality of vials (50) to be moved in a processing line, comprising a plate (20) having a plurality of housing seats (30) for housing the vials (50) and a plurality of rigid spacer elements (40; 140) arranged within said housing seats (30) and configured to maintain the vials (50) in a suspended position with respect to a substantially horizontal operating surface (100) of the processing line, wherein each of said housing seats (30) comprises an open base (32) intended to face towards said operating surface (100), wherein said base (32) has a predetermined thickness (W) and is distinct from said spacer elements (40; 140).

2. The holder device (10) according to claim 1, wherein said base (32) defines a first support plane (P1) for supporting the holder device (10) on said operating surface (100) and wherein at least one of said spacer elements (40; 140) defines, within the housing seat (30), a second support plane (P2) for supporting a vial (50), said second support plane (P2) being parallel to said first support plane (P1) and spaced apart from said first support plane (P1) by a predetermined distance (D).

3. The holder device (10) according to claim 2, wherein said predetermined distance (D) is greater than three times said predetermined thickness (W).

4. The holder device (10) according to claim 2 or 3, wherein each housing seat (30) comprises a tubular wall (34) extending from said base (32) along a substantially vertical longitudinal axis (Y) and at least three spacer elements (40) radially protruding from said tubular wall (34) towards the inside of said housing seat (30) and circumferentially equally spaced from each other.

5. The holder device (10) according to claim 4, wherein said spacer elements (40) are formed in a single piece with said tubular wall (34).

6. The holder device (10) according to claim 4 or 5, wherein said base (32) is defined by at least three support elements (32a) radially protruding from said tubular wall (34) towards the inside of said housing seat (30) and circumferentially equally spaced from each other, wherein each spacer element (40) is arranged above a respective support element (32a).

7. The holder device (10) according to claim 6, wherein said spacer elements (40) have a circumferential extension smaller than that of said support elements (32a), an axial extension larger than that of said support elements (32a) and a radial extension smaller than that of said support elements (32a).

8. The holder device (10) according to any one of claims 4 to 7, wherein said housing seats (30) extend on opposite sides with respect to said plate (20), and wherein the tubular walls (34) of some pairs of adjacent housing seats (30) comprise, on the opposite side to the respective base (32) with respect to said plate (20), respective lateral through openings (35) mutually facing.

9. The holder device (10) according to any one of claims 4 to 8, wherein each housing seat (30) comprises at least three longitudinal ribs (38) radially protruding from said tubular wall (34) towards the inside of said housing seat (30), each of said at least three spacer elements (40) being an extension of a respective longitudinal rib (38).

10. The holder device (10) according to any one of the previous claims, wherein said spacer elements (40; 140) are made of a material that is at least partially thermally conductive or non-thermally insulating.

11. An organised assembly (150) of vials (50), comprising a holder device (10) according to any one of the previous claims and a plurality of vials (50) having respective base surfaces (52) resting on the spacer elements (40; 140)of respective housing seats (30).

12. A container (200) for housing an organised assembly (150) of vials (50) according to claim 11, wherein each of said vials (50) contains a freeze-dried product.
